# EUROPEAN PATENT APPLICATION

(11) **EP 3 733 229 A1**
(43) Date of publication of application: **04.11.2020**
(21) Application number: 20178671.2
(22) Date of filing: 10.10.2016
(51) Int. Cl.: A61M 5/24, A61M 5/31

(54) **MEDICAMENT DELIVERY VISUAL USER INDICATOR**

(30) Priority: 21.01.2016 US 201662281536 P; 07.07.2016 US 201615204542; 19.09.2016 US 201615269248
(62) Divisional of application: 16784694.8
(71) Applicant: West Pharma Services IL, Ltd., 4366411 Ra'anana (IL)
(72) Inventor: Cabiri, Oz, 4526611 Hod Hasharon (IL); Hezkiahu, Ran, 4668346 Herzliya (IL)
(74) Representative: Petraz, Gilberto Luigi

(57) **Abstract**

Disclosed herein is a pharmaceutical delivery device comprising a cartridge containing a fluid, the cartridge including a light pipe section which acts as a light pipe having an input location; and a light diffusing section optically coupled to the light pipe; and a light source optically coupled to the cartridge at the input location to the light pipe, such that at least some light from the light source entering at the input location is diffused at the light diffusing section.

## Description

### FIELD AND BACKGROUND OF THE INVENTION

The present invention, in some embodiments thereof, relates to a visual user indicator and, more particularly, but not exclusively, to a medicament delivery device used as a user indicator light.

U.S. Patent No. US8784378 discloses "A visual identification coding for a cartridge or cartridge holder for use with a drug delivery device is described. The visual identification coding includes a cartridge containing a drug and a light source located on the cartridge to visually indicate that the correct cartridge has been inserted into the drug delivery device. The light source may be a light emitting diode (LED), a surface mount diode (SMD), or an organic light emitting diode (OLED). A cartridge holder may also be included to receive the cartridge. In another embodiment, the light source may be located on the cartridge holder or a dose setting member."

U.S. Patent Application Publication no. 2014/0228768 to Eggert discloses "a handheld medical device having a housing, at least one operator activatable button mounted on a surface of the housing, and a light source mounted within the housing below the button and arranged to direct light towards the button. Substantially the whole of the button and the surface of the housing adjacent the button are opaque, save for a narrow strip adjacent the periphery of the button which is non-opaque."

Additional background art includes International Patent Application Publication no. WO2013173092 to the present inventor.

### SUMMARY OF THE INVENTION

*Example 1:* A medical delivery device having a void for containing a cartridge and at least one window, comprising: a cartridge containing a fluid material, the cartridge including: a light pipe section which acts as a light pipe having an input location; and a light diffusing section optically coupled to the light pipe and aligned with the window when the cartridge is positioned in the void; a light source optically coupled to the cartridge at the input location to the light pipe, such that at least some light from the light source entering at the input location is diffused at the light diffusing section.
*Example 2:* A medical delivery device, comprising: a housing defining a void therein for a cartridge and having a window overlapping with at least part of the void; a cartridge containing a fluid material and positioned within the void, the cartridge including: a light pipe section which acts as a light pipe having an input location; and a light diffusing section optically coupled to the light pipe and aligned with the window when the cartridge is positioned in the void; a light source positioned within the housing and optically coupled to the cartridge at the input location to the light pipe, such that at least some light from the light source entering at the input location is diffused at the light diffusing section and exits the housing through the window.
*Example 3:* The device according to any of examples 1-2, further comprising a housing defining a void therein for the cartridge and having a window overlapping with at least part of the void; wherein the cartridge is positioned within the void, and the light source is positioned within the housing, such that the light diffused at the light diffusing section exits the housing through the window.
*Example 4:* The device according to any of examples 1-3, further comprising a control circuitry having instructions to operate the light source in accordance with an operation status of the medical delivery device.
*Example 5:* The device according to any of examples 1-2, wherein the light diffusing section comprises at least one protruding element.
*Example 6:* The device according to example 5, wherein the protruding element at least partially surrounds a circumference of the cartridge.
*Example 7:* The device according to example 5, wherein the protruding element extends at an angle beyond a tangent to a wall of the cartridge.
*Example 8:* The device according to example 1, wherein the light diffusing section comprises a surface area having increased surface roughness.
*Example 9:* The device according to example 8, wherein the surface area diffuses the light in an even distribution.
*Example 10:* The device according to example 8, wherein the surface area is sized to distribute the light over an area at least twice an area defined by a surface area of the light source.
*Example 11:* The device according to example 1, wherein the light diffusing section comprises at least one bulge.
*Example 12:* The device according to example 1, wherein the light diffusing section comprises at least one slit.
*Example 13:* The device according to example 1, wherein the cartridge further comprises indicia.
*Example 14:* The device according to example 13, wherein the light diffusing section is positioned in proximity to the indicia, such that the light pipe and the indicia are viewable from a single viewing direction.
*Example 15:* The device according to example 1, wherein the light source is provided at a number having a range of 2 to 7 light sources.
*Example 16:* The device according to example 1, wherein the light source is provided at a number having a range of 3 to 6 light sources.
*Example* 17: The device according to example 16, having a plurality of the input locations and a plurality of the light diffusing sections, each of which is coupled to each of the input locations.
*Example 18:* The device according to example 17, wherein each of the input locations and its associated light diffusing section are optically coupled to single light source of the light sources.
*Example 19:* A method for indicating a user about a state of a medical delivery device comprising: optically coupling at least one light source with a cartridge containing a fluid material and having a light input location; optically coupling through the cartridge a light diffusing section to the light input location; positioning the at least one light source and the cartridge in a housing having a window, such that the light diffusing section is aligned with the window; operating the at least one light source in accordance with a state of the medical delivery device.
*Example 20:* The method according to example 19, wherein the state of the medical delivery device comprises an ongoing delivery state.
*Example 21:* The method according to example 19, wherein the state of the medical delivery device comprises a stop delivery state.
*Example 22:* The method according to example 19, wherein the state of the medical delivery device comprises an on or off state.
*Example 23:* The method according to example 19, wherein the state of the medical delivery device comprises device malfunction state.
*Example 24:* The method according to example 19, wherein the state of the medical delivery device comprises an inventory status of the fluid material.
*Example 25:* The method according to example 19, wherein the operating the at least one light source comprises turning the light source in a continuous manner.
*Example 26:* The method according to example 19, wherein the operating the at least one light source comprises turning the light source on in an intermittent manner.
*Example 27:* The method according to example 26, wherein the operating the at least one light source comprises changing a frequency of the intermittent manner.
*Example 28:* The method according to example 19, wherein the operating the at least one light source comprises turning the light source off.
*Example 29:* The method according to example 19, wherein the operating the at least one light source comprises changing the light source color.
*Example 30:* A process of manufacturing a medical delivery device, comprising: molding a cartridge to include: a transparent section which acts as a light pipe having an input location; and a light diffusing section optically coupled to the light pipe; filling the cartridge with a fluid material; providing a housing defining a void therein for a cartridge and having a window overlapping with at least part of the void; positioning the cartridge within the void such that the light diffusing section is aligned with the window; positioning at least one light source within the housing and optically coupling the at least one light source to the light pipe through the input location, such that at least some light from the light source entering at the light pipe input is diffused at the light diffusing section and exits the housing through the window.
*Example 31:* The process according to example 30, wherein the molding comprises using Crystal Zenith.
*Example 32:* The process according to any of examples 30-31, further comprising operatively coupling the at least one light source with a control circuitry.
*Example 33:* The process according to example 32, further comprising programming the control circuitry to turn on or turn off the at least one light source according to an operation state of the device.
*Example 34:* The process according to example 32, further comprising programming the control circuitry to adjust a light generated by the at least one light source according to an inventory status of the fluid material.
*Example 35:* The process according to any of examples 30-34, further comprising molding the light diffusing section in a position allowing view of a physical indicator of an inventory state of the fluid material.
*Example 36:* The process according to example 35, wherein the physical indicator comprises a fluid level of the fluid material.
*Example 37:* The process according to example 35, wherein the physical indicator comprises indicia markings.
*Example 38:* The process according to example 35, wherein the physical indicator comprises a cartridge plunger position.
*Example 39:* A process of manufacturing a medical delivery device, comprising: molding a cartridge to include: a transparent section which acts as a light pipe having an input location; and a light diffusing section optically coupled to the light pipe; and filling the cartridge with a fluid material.
*Example 40:* The process according to example 30, further comprising: providing a housing defining a void therein for a cartridge and having a window overlapping with at least part of the void; positioning the cartridge within the void such that the light diffusing section is aligned with the window; positioning at least one light source within the housing and optically coupling the at least one light source to the light pipe through the input location, such that at least some light from the light source entering at the light pipe input is diffused at the light diffusing section and exits the housing through the window.
*Example 41:* The device according to example 1, further comprising a housing defining a void therein for the cartridge and having a window overlapping with at least part of the void; wherein the cartridge is positioned within the void, and the light source is positioned within the housing, such that the light diffused at the light diffusing section exits the housing through the window.
*Example 42:* The device according to example 1, further comprising a control circuitry having instructions to operate the light source in accordance with a status of the medical delivery device.
*Example 43:* The device according to example 1, wherein the light diffusing section comprises a surface area having increased surface roughness sized to distribute the light over an area at least twice an area defined by a surface area of the light source.
*Example 44:* The device according to example 1, wherein the cartridge further comprises indicia, and wherein the light diffusing section is positioned in proximity to the indicia, such that the light pipe and the indicia are viewable from a single viewing direction (possible for two windows or more).
*Example 45:* The method according to example 19, wherein the state of the medical delivery device comprises at least one of an ongoing delivery state, a stop delivery state, an on or off state, device malfunction state and any combination thereof.
*Example 46:* The method according to example 19, wherein the operating the at least one light source comprises turning the light source in a continuous manner or in an intermittent manner.
*Example 47:* The process according to example 30, further comprising operatively coupling the at least one light source with a control circuitry and programming the control circuitry to operate the at least one light source according to a state of the device.
*Example 48:* The process according to example 30, further comprising programming the control circuitry to adjust a light generated by the at least one light source according to an inventory status of the fluid material.
*Example 49:* The process according to example 30, further comprising molding the light diffusing section in a position allowing view of a physical indicator of an inventory state of the fluid material.
*Example 50:* A medical delivery device having a void for containing a cartridge and at least one window, comprising:
   (a) a cartridge containing a fluid material, wherein at least part of the cartridge is lined with a layer, the layer acts as a light pipe having a light input location, and the layer further comprises a light diffusing section optically coupled to the light pipe and aligned with the window when the cartridge is positioned in the void; and
   (b) a light source optically coupled to the layer at the input location to the light pipe, such that at least some light from the light source entering at the input location is diffused at the light diffusing section.
*Example 51:* The device according to example 50, wherein at least a portion of the layer comprises an adhesive surface for attaching onto a surface of the cartridge.
*Example 52:* The device according to example 50, wherein the layer is at least partially transparent.
*Example 53:* The device according to example 52, wherein the light diffusing section comprises at least one marking, the marking diffuses a limited range of wavelengths.
*Example 54:* The method according to Example 53, wherein the marking is text directed to a user indication of the device.
*Example 55:* The device according to Example 50, wherein the layer is configured to diffuse light having a wavelength selected according to at least one visual perceptive property of said fluid.
*Example 56:* A pharmaceutical delivery device comprising: a cartridge containing a fluid, the cartridge including: a light pipe section which acts as a light pipe having an input location; and a light diffusing section optically coupled to the light pipe; and a light source optically coupled to the cartridge at the input location to the light pipe, such that at least some light from the light source entering at the input location is diffused at the light diffusing section.
*Example 57:* The device according to Example 1, further comprising a housing defining a void therein for the cartridge and having at least one window, at least one of which overlapping with at least part of the void; wherein the cartridge is positioned within the void and the light source is positioned within the housing, such that the at least some light which is diffused at the light diffusing section, exits the housing through the window.
*Example 58:* The device according to any of Examples 1, 19, 30, 50, 56 and 57, wherein the fluid is a bioactive material.
*Example 59:* The device according to any of Examples 56-58, further comprising a control circuitry having instructions to operate the light source in accordance with a status of the pharmaceutical delivery device.
*Example 60:* The device according to any of Examples 56-59, wherein the light diffusing section comprises at least one protruding element.
*Example 61:* The device according to Example 60, wherein the protruding element at least partially surrounds a circumference of the cartridge.
*Example 62:* The device according to Example 60, wherein the protruding element extends at an angle beyond a tangent to a wall of the cartridge.
*Example 63:* The device according to any of Examples 56-62, wherein the light diffusing section comprises a surface area having increased surface roughness sized to distribute the light over an area at least twice an area defined by a surface area of the light source.
*Example 64:* The device according to any of Examples 56-63, wherein the cartridge further comprises indicia, and wherein the light diffusing section is positioned in proximity to the indicia, such that the light pipe and the indicia are viewable from a single viewing direction.
*Example 65:* The device according to any of Examples 56-64, wherein the light source is provided at a number having a range of 2 to 7 light sources.
*Example 66:* The device according to any of examples 56-65, wherein the light diffusing section is the cartridge wall.
*Example 67:* The device according to any of Examples 56-11, wherein the light diffusing section transmits 80% of the light from the light source.
*Example 68:* The device according to any of Examples 56-57, wherein the light diffusing section is a plunger provided in the cartridge.
*Example 69:* A method for indicating a user about a state of a pharmaceutical delivery device comprising: optically coupling at least one light source with a cartridge containing a fluid and having a light input location; optically coupling through the cartridge a light diffusing section to the light input location; positioning the at least one light source and the cartridge in a housing having at least one window, such that the light diffusing section is aligned with the at least one window; and operating the at least one light source in accordance with a state of the pharmaceutical delivery device.
*Example 70:* The method according to Example 69, wherein the state of the pharmaceutical delivery device comprises at least one of an ongoing delivery state, a stop delivery state, an on or off state, device malfunction state and any combination thereof.
*Example* 71: The method according to Example 69, wherein the state of the pharmaceutical delivery device comprises an inventory status of the fluid.
*Example 72:* The method according to any of Examples 69-71, wherein the operating the at least one light source comprises turning the light source in a continuous manner or in an intermittent manner.
*Example 73:* The method according to any of Examples 69-72, wherein the operating the at least one light source comprises changing the light source color.
*Example 74:* A process of manufacturing a pharmaceutical delivery device, comprising: forming a cartridge to include: a transparent section which acts as a light pipe having an input location; and a light diffusing section optically coupled to the light pipe; and filling the cartridge with a fluid.
*Example 75:* The process according to Example 19, further comprising: providing a housing defining a void therein for a cartridge and having a window overlapping with at least part of the void; positioning the cartridge within the void such that the light diffusing section is aligned with the window; positioning at least one light source within the housing and optically coupling the at least one light source to the light pipe through the input location, such that at least some light from the light source entering at the light pipe input is diffused at the light diffusing section and exits the housing through the window.
*Example 76:* The process according to Example 74, wherein the forming comprises molding.
*Example* 77: The process according to Example 76, wherein the molding comprises using Crystal Zenith.
*Example 78:* The process according to any of Examples 74-77, further comprising operatively coupling the at least one light source with a control circuitry and programming the control circuitry to operate the at least one light source according to a state of the device.
*Example 79:* The process according to Example 78, further comprising programming the control circuitry to adjust a light generated by the at least one light source according to an inventory status of the fluid.
*Example 80:* The process according to any of Examples 74-79, further comprising forming the light diffusing section in a position allowing view of a physical indicator of an inventory state of the fluid.
*Example 81:* A pharmaceutical delivery device having a void for containing a cartridge and at least one window, comprising: a cartridge containing a fluid, wherein at least part of the cartridge is lined with a layer, the layer acts as a light pipe having a light input location, and the layer further comprises a light diffusing section optically coupled to the light pipe and aligned with the window when the cartridge is positioned in the void; and a light source optically coupled to the layer at the input location to the light pipe, such that at least some light from the light source entering at the input location is diffused at the light diffusing section.
*Example 82:* The device according to Example 81, wherein at least a portion of the layer comprises an adhesive surface for attaching onto a surface of the cartridge.
*Example 83:* The device according to any of Examples 81-82, wherein the layer is at least partially transparent.
*Example 84:* The device according to Example 83, wherein the light diffusing section comprises at least one marking, the marking diffuses a limited range of wavelengths.
*Example 85:* The method according to Example 84, wherein the marking is text directed to a user indication of the device.
*Example 86:* The device according to any of Examples 81-85, wherein the layer is configured to diffuse light having a wavelength selected according to at least one visual perceptive property of the fluid.
*Example 87:* A cartridge for positioning within a pharmaceutical delivery device having a housing, the cartridge comprising: a light pipe section which acts as a light pipe having an input location; and a light diffusing section optically coupled to the light pipe; wherein the light diffusing section extends beyond a plane defined by the housing.
*Example 88:* The cartridge according to Example 87, wherein when the input location is optically coupled to a light source, at least some light from the light source entering at the input location is diffused at the light diffusing section.
*Example 89:* The cartridge according to any of Examples 87-88, wherein the cartridge and the light diffusing section are made from the same material.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

### BRIEF DESCRIPTION OF THE SEVERAL VIEWS OF THE DRAWING

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is block diagram illustrating an exemplary device having a user indicator light in accordance with some embodiments of the current invention;
FIG. 2 schematically illustrates a top view of an exemplary drug delivery device having a user indicator light in accordance with some embodiments of the current invention;
FIGs. 3A-B schematically illustrate an exemplary drug delivery device housing in accordance with some embodiments of the current invention, wherein FIG. 3A illustrates a perspective view of a housing cross section and FIG. 3B illustrates a perspective view of a housing cross section and an optional cartridge in accordance with some embodiments of the current invention;
FIG. 4 schematically illustrates a perspective view of an exemplary cartridge having an optional extended light diffusing section, in accordance with some embodiments of the current invention;
FIGs. 5A-B schematically illustrates a perspective view of an exemplary cartridge having an optional light diffusing surface and/or sticker, in accordance with some embodiments of the current invention, wherein FIG. 5A illustrates a light diffusing surface and FIG. 5B illustrates a light diffusing sticker;
FIGs. 6A-B schematically illustrate a perspective view of an exemplary cartridge having optional light diffusing protrusions, in accordance with some embodiments of the current invention, wherein FIG. 6A illustrates protrusions arranged equidistantly, and FIG. 6B illustrates protrusions arranged in a non-equidistant manner;
FIG. 7 schematically illustrates a perspective view of an exemplary cartridge having an optional light diffusing bulge, in accordance with some embodiments of the current invention;
FIG. 8 schematically illustrates a perspective view of an exemplary cartridge having an optional light diffusing slot, in accordance with some embodiments of the current invention;
FIG. 9 schematically illustrates a perspective view of an exemplary cartridge having a plunger identifiable by alight diffusing surface, in accordance with some embodiments of the current invention;
FIG. 10 is a flow chart illustrating an exemplary manufacturing process, in accordance with some embodiments of the current invention;
FIG. 11 is a flow chart illustrating an exemplary light indication algorithm, in accordance with some embodiments of the current invention; and
FIG. 12 is a flow chart illustrating an exemplary method for using a medicament cartridge as a user indicator light, in accordance with some embodiments of the current invention.

### DESCRIPTION OF SPECIFIC EMBODIMENTS OF THE INVENTION

The present invention, in some embodiments thereof, relates to a visual user indicator and, more particularly, but not exclusively, to a medicament delivery device used as a user indicator light.

### Overview

An aspect of several embodiments of the invention relates to using a cartridge as a light pipe for indicating a status of a pharmaceutical delivery device. In some embodiments, a light pipe refers to light traveling and/or diffusing and/or being transmitted and/or guided in at least a portion of a volume of a cartridge wall, the cartridge optionally containing a medicament, e.g. a bioactive material. For example, a bioactive material is any composition having a therapeutic effect on a patient's body. Alternatively, a medicament is a non-bioactive material, such as for example, placebo. In some embodiments, the cartridge comprises a light diffusing section, e.g. an element being optically coupled to the light pipe and serving to diffuse light out of the cartridge wall. Alternatively or additionally, a light diffusing section acts as a light input, collecting light form a light source and into the cartridge pipe light. Optionally, such a cartridge is being used as a user indicator module in a drug delivery device, such as for example an injector device.

As used herein, a cartridge is a compartment containing fluid, and having a needle. In some embodiments, the needle is parallel with respect to the longitudinal axis of the cartridge. Alternatively, the needle is bent with respect to the longitudinal axis of the cartridge, optionally being substantially perpendicular to it. Fluid, as used herein, refers to a liquid and/or gas used for medical purposes. In some embodiments, fluid includes a medicament, optionally a bioactive material such as a drug, or a non-bioactive material such as a placebo. In some embodiments, fluid includes a suspension, and/or a solution, and/or a mixture of miscible or immiscible liquids.

In some embodiments, a light source is optically coupled to the cartridge, wherein at least some of the light generated by the light source is transmitted and/or guided and/or diffuses and/or travels across at least a portion of the volume of the cartridge wall. In some embodiments, a light source is optically coupled to the cartridge by a light diffusing section positioned on the cartridge such that it is embedded within the injector device; optionally the light diffusing section is not viewable from outside a housing containing the cartridge and only serves as a light input location. Alternatively or additionally, a light diffusing section optically coupled to light traveling in the cartridge, is positioned on the cartridge in a position which is viewable from outside a housing containing the cartridge.

For example, light generated by the light source travels to a light diffusing section embedded in the injector device and serving as a light input, and the light input guides the light to travel through at least a portion of the volume of the cartridge. Optionally, a second light diffusing section, optionally positioned substantially opposite to the light input, guides the light traveling through the volume of the cartridge to be viewable from the opposite direction of the cartridge. Alternatively or additionally, light diffuses through the fluid contained in the cartridge.

In some embodiments, at least two windows are provided in an injector housing, optionally for allowing a user to view more than one portion of the cartridge. Optionally, at least one window allows viewing of the medicament and/or plunger. Alternatively or additionally, at least one window allows viewing of the light diffusing section. In some embodiments, the same window allows viewing of both the medicament (and/or plunger) and the light diffusing section. A potential advantage of having a window to view the medicament, through which light is also transmitted, is that light generated within the injector assists in improving the inspection of the medicament status in the injector.

In some embodiments, light is generated by the light source in accordance with a state of a delivery cartridge device. In some embodiments, state is an operation status of the device, for example an ongoing delivery state and/or a stop-delivery state and/or an end-of-delivery state and/or an on/off state of a motor and/or availability of an electricity source and/or device malfunction. Alternatively or additionally, state is an inventory status of the amount of fluid delivered and/or left in the cartridge.

A potential advantage of using a medicament cartridge to transmit a user indicator light is reducing the need to add a separate module of an indicator light to a delivery device. Reducing the number of modules in a device is likely to reduce possible malfunctions of the device. In addition, another potential advantage is an enlarged surface area exhibiting the indicator light, which is likely to be viewable from a wide range of viewing angles. Usually the delivery cartridge surface visible by a user is larger than the typical indicator light provided in a separate module, and therefore, a user may detect the indicator light with greater ease and by looking from many different angles. In some embodiments, most of the light generated within the device comes out of the cartridge, e.g. at least 90%, or at least 80 % or at least 70 %, or at least 60 %.

In some embodiments, a light source positioned in proximity to a cartridge containing a medicament serves a double role: a first role comprises being a status indicator light configured to travel through at least a portion of the cartridge wall and be in a viewing path of a user. A second role comprises lighting the cartridge content, enabling viewing of the medicament status in the cartridge, and/or viewing of indicia provided on the cartridge wall and/or viewing of a plunger position. In some embodiments, using the same window, a user can see an indication light of the status of the device operation simultaneously with observing the status of the medicament, such as for example the fluid level of the medicament, and/or the plunger position, and/or indicia markings. A potential advantage of having the same observation window for the above indication is that it gives a user a redundant view of two indicators possibly reducing the probability of misinterpretation and/or providing to the user an indicator of when there is a malfunction in one of the indicators. In some embodiments, at least two windows are provided, at least one for viewing at least the medicament and at least one for viewing at least a cartridge light guide. Optionally, both windows are positioned to enable viewing at the same time.

In some embodiments, the same light source illuminates the cartridge (potentially facilitating reading of the graduation and/or the level of medicine) and/or includes a coded status indicator (e.g. color and/or flickering). In some embodiments, light properties are suitable to a light traveling characteristic of the medicament, e.g. different light features are used if the medicament is opaque, or transparent or translucent. In some embodiments, the color of the light source is determined in accordance with a medicament color. In some embodiments, the medicament can be a material having any color. Alternatively or additionally, the medicament can have a milky and/or opaque appearance.

In some embodiments, a plunger configured for facilitating the medicament, provides a change in the total reflection of the light traveling through the cartridge wall, optionally causing light to diffuse out of the cartridge wall to a direction of a user, at the point where the plunger edge contacts the cartridge wall, providing a light indication of the plunger position. Alternatively or additionally an optical element (for example a diffuser and/or a reflector) may be added to the plunger, for example at the base (e.g. the proximal end) of the plunger. Alternatively or additionally, a wall of the plunger being in contact with the cartridge can serve as a light guide.

In some embodiments, a housing is provided having an observation window enabling a user to view a delivery cartridge serving as a light pipe embedded in the housing. Optionally, an observation window is any aperture in a housing containing a delivery cartridge and enabling a user a visual view of the cartridge. In some embodiments, the observation window is provided for a user visual detection of an indicator light embedded inside the housing and having a light path to the cartridge. Optionally, the observation window serves as a visual indicator of a light produced inside the housing traveling across a volume of the cartridge. Potentially the observation window is doubly used for the detection of an indicator light and for a user visual inspection of the cartridge content. Alternatively or additionally, the cartridge is provided with an appendage having light transmitting and/or diffusing properties. Optionally an observation window may include a pane and/or may include an open space.

In some embodiments, operation status is indicated by using a plurality of light wave properties, for example, frequency and/or amplitude and/or duration. In some embodiments, light is transmitted continuously. Alternatively or additionally, light is transmitted intermittently, for example by flickering. In some embodiments, light properties include frequency of flickering, which might increase or decrease over time. For example, flickering frequency, in some embodiments, is correlated to the delivery speed and/or is correlated with a medicament amount having been delivered.

In some embodiments, the cartridge serves as a pipe light by having at least a portion of a transparent section. As used herein, transparent relates to a material allowing at least some traveling of light therethrough. In some embodiments the cartridge comprises a light diffusing section in the form of an appended visual feature. In some embodiments, the visual feature is configured to guide (i.e. transmit) a light path through it. Alternatively or additionally, the visual feature is configured to diffuse light through it. In some embodiments, the visual feature protrudes from the surface of the cartridge, optionally made of the same material as the cartridge, optionally molded with the outer surface of the cartridge. Alternatively, the visual feature is in a different composition than the cartridge and is designed to guide and/or diffuse light around at least a partial circumference of the cartridge.

In some embodiments the visual feature comprises a plurality of protrusions, for example circumferential protruding ribs and/or segments of circumferential protruding ribs, optionally orthogonal to the longitudinal axis of the cartridge. Alternatively or additionally, the visual feature comprises a plurality of spaced apart bulges. A potential advantage of a surface having protrusions thereon is that protruding elements are likely to transmit and/or diffuse a greater amount of light with respect to a smooth surface. In some embodiments the protrusions are equidistant, probably enabling substantial uniform transmission of light. Alternatively, the protrusions are asymmetrically distributed, probably enabling more light to transmit to a predefined surface region.

Alternatively or additionally, the visual feature comprises a plurality of slots and/or grooves. Alternatively or additionally, the visual feature comprises at least one protrusion and at least one slot. Potentially, either protrusions and/or slots are configured to make light traveling through the volume of the cartridge's wall to diffuse outside of the cartridge wall. Alternatively or additionally, at least a portion of the cartridge wall comprises increased roughness, causing light to diffuse out of the cartridge wall. In some embodiments, surface textures such as bulges and/or slots and/or roughness are provided on the outer surface of the cartridge wall. Alternatively or additionally, surface textures are provided on the inner surface of the cartridge wall.

In some embodiments, protrusions are arranged in a group, guiding a light path from an individually operated light source. Optionally, a plurality of such groups is provided, each being coupled to a distinctly operated light source. A potential advantage of multiple individually operated groups is the increase of visual information provided to the user simultaneously, by having a different interpretation assigned to each group of protrusions. For example, a first group of protrusions is linked to a first light source indicating, e.g., the delivery operation state of the device, and a second group of protrusions is linked to a second light source indicating e.g., the state of the medicament amount.

In some embodiments the visual feature comprises a projecting element having light guiding and/or diffusing properties. In some embodiments, the element is projected beyond a plane defined by the cartridge and/or the housing. A potential advantage of a light transmitting projecting element is its likelihood to better guide a propagation of light through it. In addition, a protruding element is likely to be seen and/or noticed more conveniently from a variety of observation angles.

In some embodiments, a projecting element includes a light diffusing section configured to extend beyond a plane defined by the housing of cartridge and/or a plane defined by the inner edge of a window and/or a plane defined by an outer edge of a window. A potential advantage of extending beyond the housing and/or the edge of the window is an increase in a user available viewing angles of the light diffusing section. In some embodiments, more than one light diffusing section is provided, optionally each being coupled to a different light source.

In some embodiments the cartridge comprises at least one alignment guide defining an orientation of the cartridge with respect to an embedding injector device. In some embodiments, the alignment guide projects beyond the outer surface of the cartridge. Alternatively, the alignment guide comprises a recess in the outer surface of the cartridge. Optionally, the alignment guide interlocks with a complementary geometrical feature within the injector device, optionally preventing rotation of the cartridge with respect to the device. Potentially, alignment guides enable positioning of the cartridge at a predefined orientation, for example with respect to the observation window of the device housing and/or a light source, optionally allowing an appended visual feature to be visible to a user.

An aspect of several embodiments of the invention relates to a process of manufacturing a light transmitting and/or diffusing cartridge for medicament delivery to serve as a user indicator module, used for example in an injector device.

In some embodiments, a drug delivery device is provided by embedding a cartridge in a housing having a window at least partially aligned with the cartridge position. Optionally, the window follows a cartridge's longitudinal axis, or any axis across which a medicament is expelled.

In some embodiments, at least one light source is positioned within said housing such that it is optically coupled to the cartridge. In some embodiments a range of 1 to 10 light sources is provided. Alternatively a range of 2 to 7 light sources is provided. Alternatively, a range of 3 to 6 light sources are provided. Alternatively 4 light sources are provided.

In some embodiments a cartridge is manufactured from a material which permits traveling of electromagnetic waves in a visible frequency range. Optionally, the material is at least partially transparent, optionally providing view of the composition being delivered. Alternatively, the cartridge is internally coated with an opaque material, such as for example in the case of a light-sensitive medicament, but the cartridge wall enables visible light to diffuse across its volume. In some embodiments, at least part of the cartridge is manufactured using Crystal Zenith, optionally by molding. Alternatively or additionally, at least part of the cartridge is manufactured using glass. Alternatively or additionally, at least part of the cartridge is manufactured using a standard material known in the art to be used for cartridges.

In some embodiments the cartridge is molded asymmetrically, optionally having transmitting/diffusing elements where an observation window is designed to be fit with respect to the cartridge. Alternatively or additionally, the cartridge is molded having transmitting/diffusing elements which are embedded inside the injector housing, optionally not viewable from the outside. Alternatively or additionally, the cartridge is molded to have a defined angle with respect to the observation window and housing plane.

In some embodiments, the cartridge is manufactured from layers having at least two materials. Optionally, at least one of the layering materials is opaque. Alternatively or additionally, at least one of the layering materials is transparent, optionally partially. In some embodiments, in at least one of the layers visible light travels and/or diffuses throughout its volume.

An aspect of some embodiments of the invention relates to a light guiding liner and/or sticker sized and shaped to line at least a portion of a medicament cartridge. In some embodiments, the sticker comprises a material having light diffusing properties, i.e. light can travel and/or diffuse and/or be transmitted through at least a portion of the volume of the sticker. Optionally, at least a portion of the sticker surface comprises an adhesive material for adhering onto a cartridge inner and/or outer surface. In some embodiments, the sticker includes text and/or markings over it surface, optionally visible only when being exposed to light being in a particular wavelength range.

Optionally, the sticker is visible only when sharing a visual path with a light having a wavelength in the range of 400-450, and/or 450-480, and/or 480-490 and/or 490-500, and/or 500-560, and/or 560-580, and/or 580-600, and/or 600-650 and/or 650-750, and/or any range larger, smaller or intermediate range.

In some embodiments, the text and/or markings comprise a light diffusing material and are lit once light travels to them from a light input location. Optionally, the sticker comprises a light input located inside an injector housing, and sharing a visual path with at least one light source embedded within the housing. Alternatively or additionally, text and/or markings are visible by having only a contour which is a light guiding material. Alternatively or additionally, the text and/or markings are visible by being surrounded by a light guiding sticker and not guiding light themselves, such as by punching the text and/or markings out of the sticker and creating a material-free space being in the shape of the text and/or markings.

In some embodiments, the cartridge and/or syringe is manufactured in a process distinct from the filling of the fluid reservoir. Accordingly, optionally, some light guide types are provided in the first process and other light guide types are provided in the second process. For example, molded light guide features may be provided through the molding process of the cartridge, while the liner may be assembled onto the cartridge by a different manufacturer, either before or after filling and/or sealing with the plunger. In some embodiments, the light guide features provided by the first process are configured to be compatible to a variety of light sources and/or fluids. Alternatively or additionally, the light guide features provided by the second process are specifically tailored to the specific fluid type and/or light properties of the fluid being used for filling. Optionally, the features in the first process are provided independently from the features in the second process.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Exemplary user indicator light path for use in a drug delivery device

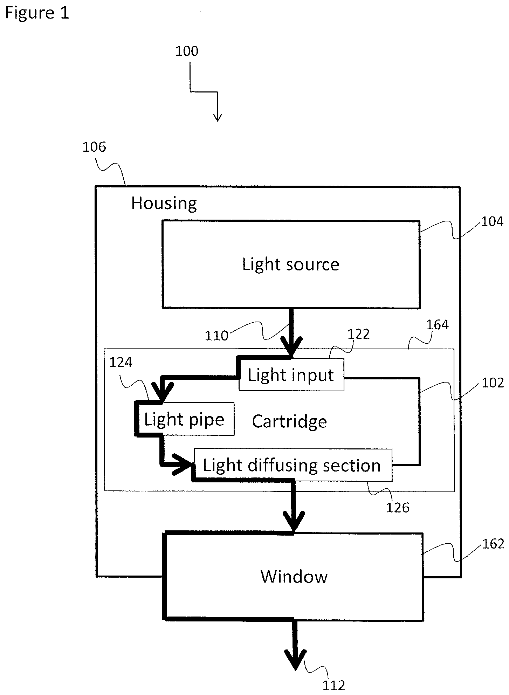

Referring now to the drawings, *FIG.* 1 illustrates a block diagram illustrating an exemplary device having a user indicator light in accordance with some embodiments of the current invention. In some embodiments, a drug delivery device is provided with a housing **106** having a window **162,** and having a void **164.** In some embodiments, a cartridge **102** containing a medicament, e.g. a bioactive material, is positioned within void **164.** In some embodiments, a light source **104** is also positioned within the housing, optionally in proximity to the cartridge.

In some embodiments, cartridge **102** is configured to serve as a light pipe, bridging light generated by light source **104** to window **162.** Optionally, cartridge **102** comprises a light input location **122** sharing an optical pathway with the light source **104.** In some embodiments, cartridge **102** is made of a material having light propagating properties. Optionally at least a portion of the wall of cartridge **102,** serves as a light pipe **124** for transmitting light at least through a portion of its volume.

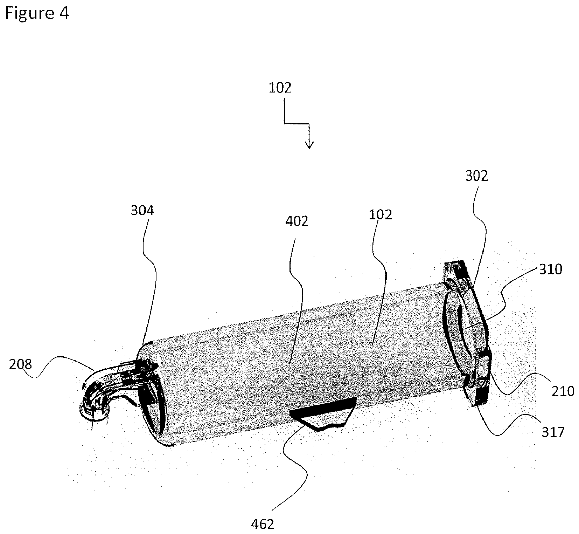

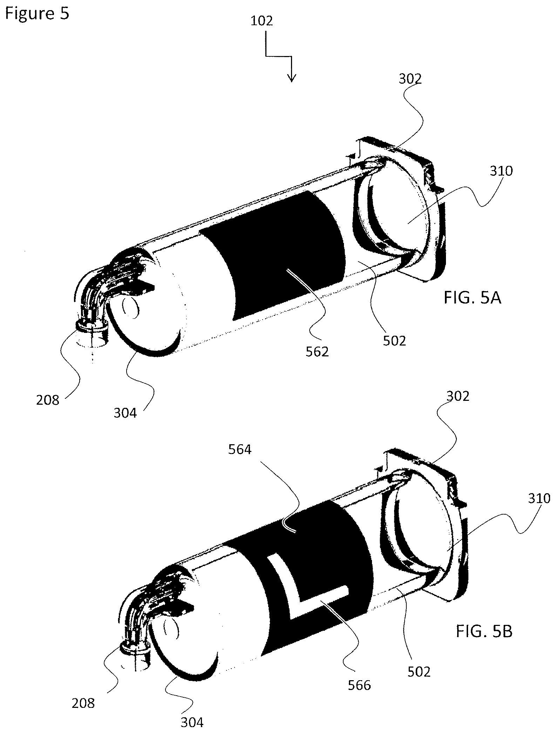

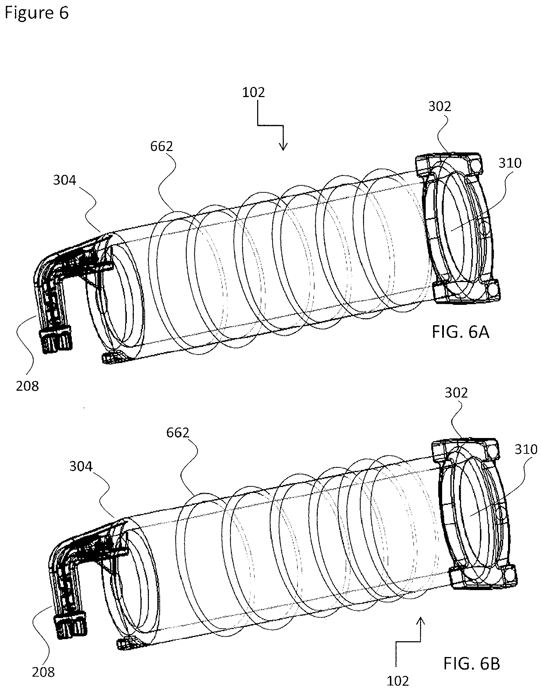

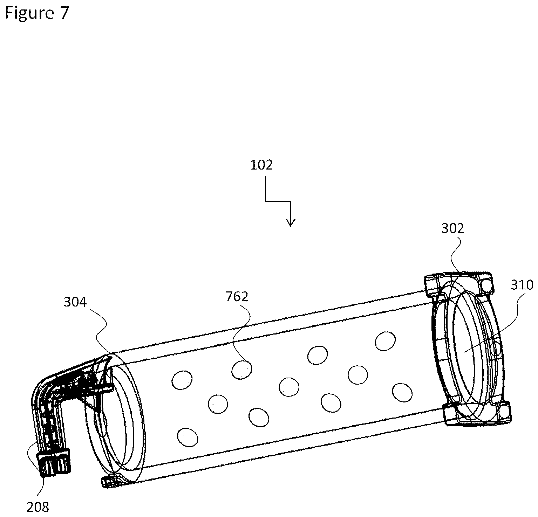

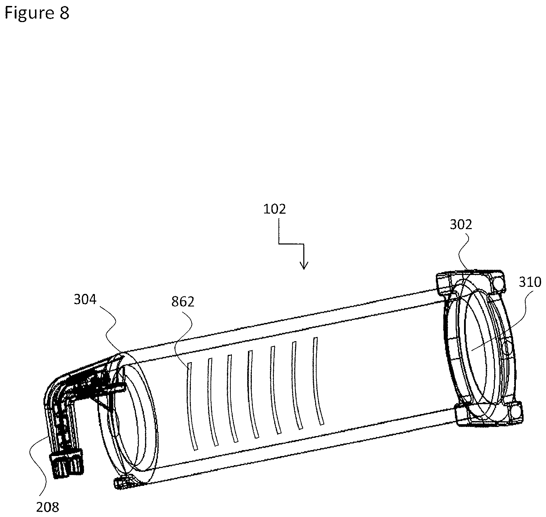

In some embodiments, cartridge **102** comprises a light diffusing section **126.** Optionally, light diffusing section **126** comprises an extending element. Optionally, the extending element is substantially perpendicular to a longitudinal axis of cartridge **102,** as shown for example in *FIG.* 4. Alternatively or additionally, light diffusing section **126** comprises a plurality of protruding elements as shown for example in *FIG. 6*. Alternatively or additionally, light diffusing section **126** comprises at least one bulge as shown for example in *FIG.* 7. Alternatively or additionally, light diffusing section **126** comprises at least one slot as shown for example in *FIG. 8**.* Alternatively or additionally, light diffusing section **126** comprises at least one surface area having an increased roughness relative to other portions of the cartridge **102** as shown for example in *FIG. 5*. In some embodiments, the distal portion of the cartridge comprises a flange which serves as a light diffusing section **126,** as shown in *FIG. 4**.*

In some embodiments, light diffusing section **126** comprises an arrangement along a longitudinal axis of the cartridge. Alternatively or additionally, light diffusing section **126** comprises an arrangement along at least a portion of the cartridge circumference. In some embodiments, light pipe **124** comprises a material extending along at least a portion of the cartridge **102** circumference, serving as a light pipe between a light source positioned in proximity to one portion of the light pipe, to a light diffusing section positioned in proximity to a second portion of the light pipe.

In some embodiments, light generated by light source **104** travels through light path **110.** Optionally, light generated by light source **104** travels to light input **122** found in cartridge **102.** In some embodiments, light travels from light input **122** into the light pipe **124** of cartridge **102.** Optionally, light pipe **124** includes a transparent section of cartridge **102.** In some embodiments, light pipe **124** comprises the entire volume of the wall of cartridge **102.** Alternatively, only a portion of cartridge **102** is made of a material enabling light traveling. In some embodiments, cartridge **102** comprises layers of materials, at least one of which allows light to travel through it.

In some embodiments, light traveling through light path **110** is internally reflected except for when encountering light diffusing section **126.** In some embodiments, light diffusing section **126** is shaped to allow light traveling through light path **110** to diffuse out of the internal volume of cartridge **102.**

In some embodiments, window **162** is aligned with cartridge **102** such that light diffusing section **126** is viewable through window **162.** As such, in some embodiments, light traveling through light path **110** diffuses out of light diffusing section **126** and out **112** of housing **106.**

In some embodiments, window **162** is positioned in a top cover of housing 106, optionally near the position of an operation button. Alternatively or additionally, window **162** is positioned in a base of housing **162,** optionally near a position of a removable safety liner.

In some embodiments, window **162** is shaped as a circle. Alternatively or additionally, window **162** is shaped as an ellipse or an elongated polygon, optionally aligned with a longitudinal axis of cartridge **102.** Alternatively or additionally, window **162** is shaped as a slot.

In some embodiments, housing **106** comprises a plurality of windows **162,** optionally aligned with different segments of cartridge **102.** Alternatively or additionally, window **162** includes a viewing section in-between a plurality of housing compartments. For example, if housing **106** is provided in a multi-part configuration, the plurality of housing parts are optionally assembled such that at least a portion of cartridge **102** can be viewed in between the housing parts. Optionally, cartridge **102** serves as a connector between pluralities of housing parts, for example, by having at least one housing for a proximal end of the cartridge and a separate at least one housing for the distal end of the cartridge, while at least a portion of the middle section of the cartridge is available for viewing in between the proximal and distal housing compartments.

### Exemplary drug delivery device having a cartridge used as a light pipe

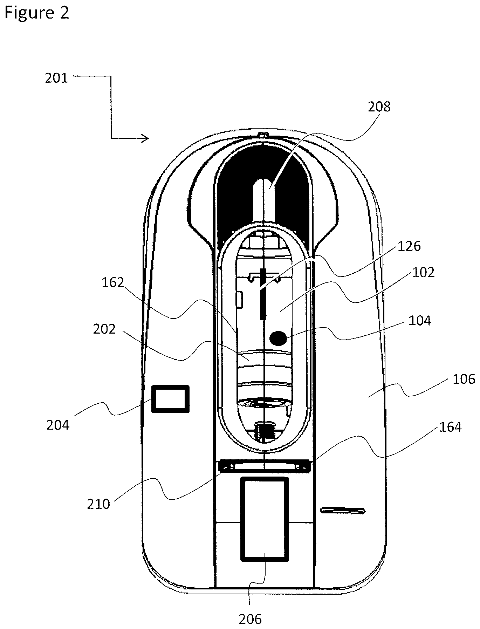

Reference is now made to *FIG.* 2, schematically illustrating a top view of an exemplary drug delivery device having a user indicator light in accordance with some embodiments of the current invention. In some embodiments, a cartridge **102** is used as a user indicator of a drug delivery device, for example injector **201.** According to some embodiments, injector **201** comprises a housing **106** having a window **162,** aligned with a cartridge **102.** The cartridge **102,** in some embodiments, is positioned such that a light diffusing section **126** is viewable through window **162.** In some embodiments, light diffusing section **126** diffuses light, originally transmitted from cartridge **102,** which serves as a light pipe from light source **104.**

In some embodiments, light diffusing section **126** is found on the dorsal side of the cartridge, i.e. the side that faces window **162.** In such embodiments, light diffusing section **126** is optionally used to transmit light diffusing through the walls of cartridge **102.** Alternatively or additionally, light diffusing section is found on the ventral side of the cartridge, i.e. the side that faces the inner portion of injector **201.** In such embodiments, light diffusion section **126** optionally serves as a light input, collecting light generated inside injector **201** by light source **104, and** transmitting the light to the cartridge walls, a light which is then viewable through window **162.** In some embodiments, window **162** comprises a dome, e.g. a curved material and/or cylindrically shaped cover, for covering and/or protecting the cartridge. A potential advantage of providing a curved covering is in protecting light diffusion section **126** if it extends beyond the surface of housing **106.** Typically, a protective cover is useful when the cartridge and/or light diffusing section **126** are made of glass and prone to break if receiving a shock. In some embodiments, the dome projects from a plane defined by the surface of the housing by at least 0.5 mm, and/or at least 1 mm, and/or at least 2 mm, and/or at least 5 mm.

In some embodiments, window **162** serves a second role when allowing viewing of plunger **202,** which is configured to push a medicament out of cartridge **102** through a fluid outlet, located at location **208.** Optionally, plunger **202** is pushed by motor **206.** Alternatively or additionally, window **162** allows viewing of the medicament fluid level found in cartridge **102.** Alternatively, cartridge **102** comprises an inner layer which is opaque, for protecting light sensitive medicaments.

In some embodiments, a control circuitry **204** is provided, optionally having instructions to operate light source **104.** For example, control circuitry **204** in some embodiments can get input from motor **206** as to an operation state of the device, and optionally accordingly provide instructions for light generation by light source **104.** Alternatively or additionally, control circuitry **204** in some embodiments can get input as to a plunger **202** position, and optionally accordingly provide instructions for light generation by light source **104.**

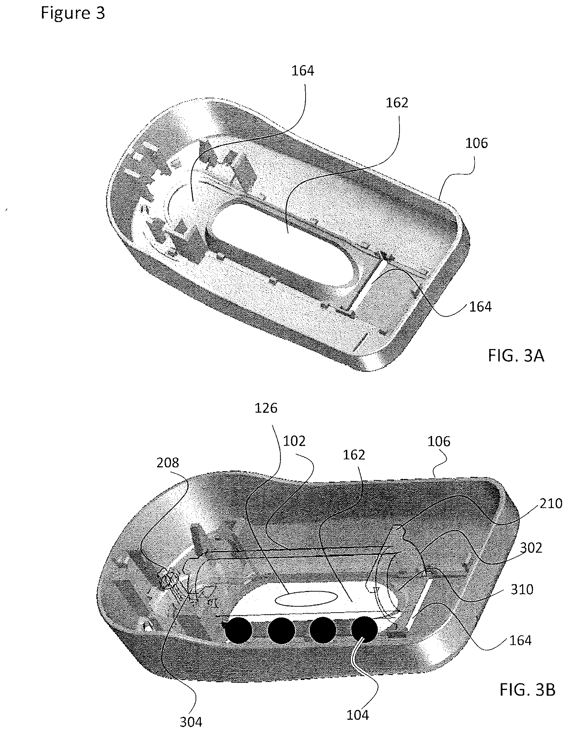

Optionally, housing **106** comprises at least two windows **162** and **164.** In some embodiments, window **164** allows viewing of the distal portion **302** of cartridge **102.** Optionally, the distal portion of cartridge **102** comprises a light diffusing section in the form of a flange **210,** which is further illustrated in detail in *FIG.* 3. In some embodiments, window **164** is sized and shaped to allow viewing of the flange, the window optionally being in the shape of a slot.

### Exemplary drug delivery device housing and cartridge

Reference is now made to *FIG.* 3, schematically illustrating an exemplary drug delivery device housing in accordance with some embodiments of the current invention, wherein *FIG. 3A* illustrates a perspective view of a housing cross section, and *FIG. 3B* illustrates a perspective view of a housing cross section and accommodating a cartridge in accordance with some embodiments of the current invention.

In some embodiments, a drug delivery device, such as for example an injector, comprises a housing **106** having a window **162.** In some embodiments, housing **106** comprises a void **164** sized and shaped to accommodate a cartridge **102.** Optionally, window **162** is provided in accordance with the void **164** such that the cartridge **102** is at least partially viewable from the window. In some embodiments, the portion of the cartridge viewable from the window is configured to serve as a user indicator light, optionally by including in the viewable portion a light diffusing section **126.**

In some embodiments, cartridge **102** comprises a proximal end **304** defined by having a needle accommodating member **208,** and a distal end **302** defined by having a bore **310** for accommodating a plunger. In some embodiments, plunger is configured to be pushed along a longitudinal axis of cartridge **102,** optionally, being aligned with the longitudinal axis of window **164.** In some embodiments, non-symmetric orientation features of the cartridge lead to positioning of the cartridge such that the distal end **302,** proximal end **304** and the light diffusing section **126** are correctly aligned with housing **106** and/or window **162.** For example, interlocking members could be provided in cartridge **102** and housing **106.** In some embodiments, such non-symmetric orientation alignment features keep cartridge **102** from shifting and/or rotating within housing **106.** Alternatively or additionally, a distal and/or proximal end of the cartridge includes a connector, such as for example a septum and/or a physical connecting feature, e.g. a snap and/or a circumferential lip.

In some embodiments, light source **104** is positioned in housing **106** such that it shares an optical pathway with at least a portion of cartridge **102,** optionally with a portion comprising a transparent section for allowing light to travel through a volume of cartridge **102,** optionally through its wall. In some embodiments, more than one light source is provided, optionally more than 2. Alternatively, more than 3 light sources are provided. Alternatively more than 4 light sources are provided. In some embodiments, each light source is optically coupled to a different light pipe provided in cartridge **102,** and/or optically coupled to a different light diffusing section. In some embodiments, a plurality of light sources is provided to enhance the viewing area of the indication light, optionally having all light sources operated in the same manner. Alternatively, a plurality of light sources is provided to allow different indications operated simultaneously, for example, a green light indicates operating status of the device motor and it is simultaneously turned on together with a blinking red light indicating delivery. In some embodiments, light source **104** comprises a light emitting diode.

### Exemplary cartridge having an extended light diffusing section

Reference is now made to *FIG. 4*, schematically illustrating a perspective view of an exemplary cartridge having at least one extended light diffusing section, in accordance with some embodiments of the current invention.

In some embodiments, light diffusing surface **462** is provided to diffuse light traveling in light pipe **402,** optionally being a wall of cartridge **102.** In some embodiments, light pipe **402** comprises the entire wall of cartridge **102** extending from distal portion **302** to proximal portion **304.** Alternatively, light pipe **402** is provided by having a transparent section only in a portion of cartridge **102,** optionally, only a portion of a longitudinal axis. Alternatively, light pipe **402** is provided by having a transparent section only in a portion of cartridge **102,** optionally, only a portion of a circumference of the cartridge **102.**

In some embodiments, light diffusing section **462** extends at an angle beyond a plane being tangent to the circumference of cartridge **102.** Alternatively or additionally, light diffusing section **462** extends to a direction perpendicular to the longitudinal axis of cartridge **102.** In some embodiments, light diffusing section **462** comprises a longitudinal axis substantially aligned with the longitudinal axis of cartridge **102.** Alternatively, light diffusing section **462** comprises a longitudinal axis substantially perpendicular to the longitudinal axis of cartridge **102.**

In some embodiments, section **462** is molded with the cartridge in a molding process. Alternatively or additionally, section **462** is added to the cartridge, optionally by providing section **462** to be sized and shaped to fit at least a portion of the cartridge. In some embodiments, section **462** is pressed against the light transmitting wall/layer of the cartridge.

Optionally, the distal end **302** of cartridge **102** comprises a flange **210.** In some embodiments, flange **210** serves as a light diffusing section, in addition to or instead of light diffusing section **462.** In some embodiments, flange **210** comprises an edge at least partially surrounding the perimeter of distal end **302,** optionally having at least one brim feature **317** extending beyond the perimeter of distal end **302** in a direction away from the central axis of cartridge **102.** Potentially, brim feature **317** can guide light generated by light source comprised in the housing, and optionally deliver the light through flange **210.** In some embodiments, at least a portion of flange **210** delivering the light is viewable from outside the housing. Alternatively or additionally, flange **210** and/or brim **317** are sized and shaped to complement at least one feature comprised in the housing, potentially fixing an orientation of the position of the cartridge within the housing.

A potential advantage of guiding light through a light diffusing section provided in the distal end **302** of cartridge **102** is that the distal end region is potentially free of medicament, which is probably located in a more proximal position of the cartridge, after the plunger. An environment without medicament potentially allows light to diffuse without interference or altering effects, which might be caused by a presence of a medicament.

### Exemplary cartridge having a light diffusing surface area

Reference is now made to *FIG. 5A*, schematically illustrating a perspective view of an exemplary cartridge having at least one light diffusing surface area, in accordance with some embodiments of the current invention.

In some embodiments, light diffusing surface area **562** is provided in cartridge **102.** In some embodiments, surface area **562** is configured to diffuse light traveling through light pipe **502** provided by cartridge **102** by having a rough surface. In some embodiments, a rough surface is defined as a surface which is not tangential to the cartridge circumference and/or extending in a variety of angles, optionally without order. In some embodiments, the greater the angles of the non-tangential surfaces, the greater the roughness level is defined. Potentially, light traveling through light pipe 502 is internally reflected when encountering a smooth surface and/or diffuses out of the light pipe when encountering rough surface 562.

In some embodiments, more than one rough surface area 562 is provided. In some embodiments, surface area **562** is configured to diffuse light in an even manner, i.e. diffuse equal portions of light per area unit. Alternatively, surface area **562** is configured to provide unequal portions of light to diffuse per area unit, for example, by having a gradient of roughness level and/or areas of greater/smaller roughness level.

Reference is now made to *FIG. 5B*, schematically illustrating an aspect of some embodiments of the invention of providing a cartridge **502** with liner and/or sticker **564.** In some embodiments, cartridge **502** is lined with sticker **564** on its inner surface. Alternatively or additionally, sticker **564** is lined over the outer surface of cartridge **564.** In some embodiments, sticker **564** comprises a material having light guiding properties, and optionally, light generated inside an injector housing shares a visual path with sticker **564.**

In some embodiments, sticker **564** comprises at least one marking **566,** which is optionally text and/or indicia and/or a symbol and/or any other visual mark. Optionally, marking **566** is only visible when a shared-path light is generated within the injector housing. In some embodiments, marking **566** is only visible when the light generated inside has a particular range of wavelengths, for example, being in the range of 400-450, and/or 450-480, and/or 480-490 and/or 490-500, and/or 500-560, and/or 560-580, and/or 580-600, and/or 600-650 and/or 650-750, and/or any range larger, smaller or intermediate range. In some embodiments, an adhesive layer and/or a sticker has a thickness in the range of about 0.05 mm to about 1 mm. Alternatively, a thickness is in a range of 0.25 mm to about 0.75 mm.

Optionally, marking **566** is visible sharing a visual path with an inner light source, while the surrounding sticker **564** does not guide light through it. Alternatively or additionally, marking **566** and surrounding sticker **564** guide light having different wavelength ranges. Alternatively or additionally, only surrounding sticker **564** guides light, while marking **566** does not, such as for example, by punching out the area being taken by and/or the outline surrounding marking **566.** In some embodiments, marking **566** is printed on the sticker before its placement onto the cartridge. In some embodiments, marking 566 comprises text, optionally directed to a user indication of the device, such as for example, indicating "OK" and/or "Error" if the device malfunctions, and/or "Cold". User indicating text is optionally provided to a user using a different color, or a different range of wavelengths, for each user indicating text. For example, "Error" may be indicated with a red light and/or provided with a red filter, "OK" may be presented as green and "Cold" may be presented with blue.

In some embodiments, sticker **564** is flexible. Optionally, sticker **564** can be lined over any commercially available cartridge. In some embodiments, sticker **564** is lined over at least a portion of a cartridge which has been manufactured by molding, after the molding process is done, optionally before cartridge **502** is filled with a medicament. Alternatively, sticker **564** is lined over cartridge **502** after the cartridge is filled. In some embodiments, at least part of sticker **564** is transparent. Alternatively or additionally, at least part of sticker **564** is semi-transparent. Alternatively or additionally, at least part of sticker **564** is opaque.

In some embodiments, sticker 564 is configured to transmit light having a particular range of wavelengths. The wavelength transmitted by sticker 564 is optionally selected according to at least one visual perceptive property of the medicament comprised in the cartridge. In some embodiments, a visual perceptive property comprises the color of the medicament. Alternatively or additionally, the visual perceptive property includes the reflectivity of the medicament. Alternatively or additionally, the visual perceptive property includes the absorbance of the medicament.

### Exemplary cartridge having light diffusing protruding elements

Reference is now made to *FIG.* 6, schematically illustrating a perspective view of an exemplary cartridge having a plurality of light diffusing protrusions, in accordance with some embodiments of the current invention, wherein *FIG. 6A* illustrates protrusions arranged equidistantly, and *FIG. 6B* illustrates protrusions arranged in a non-equidistant manner.

In some embodiments, at least one protruding element **662** is provided, extending along at least a portion of a circumference of cartridge **102.** In some embodiments, a plurality of protruding elements **662** is provided. Optionally, the protruding elements **662** are arranged equidistantly, optionally along a longitudinal axis of cartridge **102.** A potential advantage of equidistant elements **662** is a uniform view from a variety of viewing angles. Alternatively, protruding elements **662** are arranged non-equidistantly, optionally with respect to a longitudinal axis of cartridge **102.** A potential advantage of non-equidistant elements **662** is for example allowing viewing the medicament being delivered at a portion having elements **662** being more distant from each other.

### Exemplary cartridge having a light diffusing bulge

Reference is now made to *FIG.* 7, schematically illustrating a perspective view of an exemplary cartridge having at least one light diffusing bulge, in accordance with some embodiments of the current invention.

In some embodiments, light diffusing section of cartridge **102** comprises at least one bulge **762.** In some embodiments, bulge **762** comprises a protruding surface limited to a relatively small surface area. In some embodiments, a bulge **762** protrudes from a section area being in a range of about 1% to about 5% of the entire surface area of the cartridge **102.** Alternatively or additionally, a bulge **762** protrudes from a section area being in a range of about 10% to about 20% of the entire surface area of the cartridge **102.** Alternatively or additionally, a bulge **762** protrudes from a section area being in a range of about 20% to about 30% of the entire surface area of the cartridge **102.**

In some embodiments, a bulge **762** extends to a height being no more than 0.2 mm. Alternatively or additionally, bulges **762** extend to a height being no more than 0.5 mm. Alternatively or additionally, bulges **762** extend to a height being no more than 1 mm.

### Exemplary cartridge having a light diffusing slot

Reference is now made to *FIG. 8**,* schematically illustrating a perspective view of an exemplary cartridge having at least one light diffusing slot, in accordance with some embodiments of the current invention.

In some embodiments, light diffusing section comprises at least one slot **862** in cartridge **102.** In some embodiments, slots **862** are provided as etched elements in cartridge **102.** Optionally, slots **862** are provided along an entire circumference of cartridge **102.** Alternatively or additionally, slots **862** are provided along a portion of the circumference of cartridge **102.** Alternatively or additionally, slots **862** are provided being parallel to a longitudinal axis of the cartridge **102.** Optionally, longitudinally aligned slots **862** enhance the optical path of viewing the fluid inventory status of the medicament. Optionally the inner surface of a slot may be smooth and/or rough and/or having a varying roughness.

### Exemplary cartridge having a light diffusing plunger contact

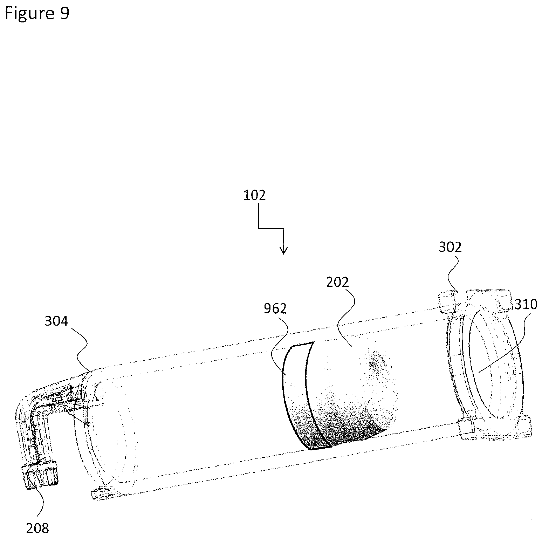

Reference is now made to *FIG.* 9, schematically illustrating a perspective view of an exemplary cartridge having a plunger identifiable by diffusing and/or reflective light, in accordance with some embodiments of the current invention.

In some embodiments, plunger **202** defines a contact wall **962** with an inner wall of cartridge **102.** In some embodiments, contact wall **962** serves as a light diffusing section by causing light to diffuse at the contact wall with plunger **202.** In some embodiments, light emitted by contact wall **962** helps to identify the location of the plunger, potentially aiding in following a delivery of a medicament. In some embodiments, the light diffusing wall is at the proximal portion of the plunger (where the plunger contacts the medicament). Locating the light diffusing section at the proximal end of the plunger has the potential advantage of showing the contact point of the medicament with the plunger. Alternatively or additionally the light diffusing wall is at the distal end of the plunger. Alternatively or additionally, the light diffusing wall is located between the distal and proximal ends of the plunger. In some embodiments, indicia are provided for gauging the volume of medicament remained based on the location of the light diffusing wall of the plunger.

### Exemplary manufacturing process

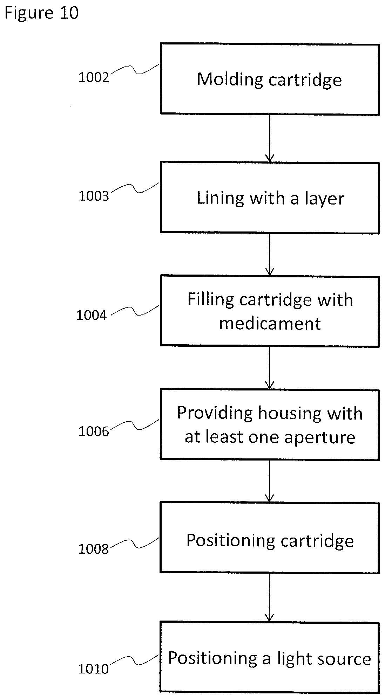

Reference is now made to *FIG. 10* showing a flow chart illustrating an exemplary manufacturing process, in accordance with some embodiments of the current invention.

In some embodiments, a process begins by molding a cartridge 1002 configured for serving as a light pipe and having a light diffusing section. Optionally, the entire cartridge is molded using a transparent material, optionally a polymer, optionally, Crystal Zenith and/or glass. Alternatively or additionally, only a portion of a cartridge is molded using transparent material, optionally the portion defines a light input location. Optionally, the molded cartridge is coated internally with a second material layer, optionally having different light propagating characteristics than the molding material.

In some embodiments, after molding the cartridge, lining of the cartridge surface is provided 1003, for example, by lining at least a portion of the cartridge surface with a sticker, optionally a sticker which serves as a light diffuser, optionally lining the inner surface of the cartridge. Alternatively or additionally, the sticker lines the outside surface of the cartridge. In some embodiments, the sticker prevents at least a portion of the light from reaching the medicament, potentially useful when the medicament used is light-sensitive. In some embodiments, the sticker comprises a reflecting material. Alternatively or additionally, the sticker is provided with written text. Alternatively or additionally, the cartridge is provided with a layer of a light guiding element, optionally by linking in a chemical fashion.

In some embodiments, a sticker which is lined over at least a portion of the cartridge contains light-sensitive illustrations and/or text. Alternatively or additionally, the sticker contains fluorescent features which are detectable only when exposed to a specific range of wavelengths. For example, the sticker contains text and/or indicia which can only be seen when the embedded light source is on and/or when a specific wavelength is emitted. Optionally, the sticker is transparent, or semi-transparent, or opaque.

In some embodiments, once molded, a cartridge is filled with a medicament 1004. Optionally, the light input location and/or the transparent light pipe, and/or the light diffusing section are manufactured according to properties of the medicament being filled. For example, if the medicament is transparent, optionally having a non-transparent portion of the cartridge to avoid light traveling in uncontrolled set ups. Alternatively, when the medicament is light-sensitive, the cartridge is optionally coated with an opaque material.

In some embodiments, a housing having a window is provided **1006.** In some embodiments, the cartridge is positioned in the housing **1008** such that the light diffusing section is aligned with the window. Optionally, the housing comprises a void sized and shaped to fit the cartridge. In some embodiments, the housing further comprises members sized and shaped to interlock with portions of the molded cartridge, such that a predefined orientation of the cartridge with respect to the housing is provided. In some embodiments, positioning of the cartridge is conducted such that at least a portion of the cartridge is viewed from the window. Optionally, the portion of the cartridge viewed from the window comprises the light diffusing section.

In some embodiments, at least one light source is positioned in the housing 1010. In some embodiments, the light source is positioned to have an optical path with at least a portion of the cartridge, optionally being a light input location.

### Exemplary manufacturing light indication algorithm

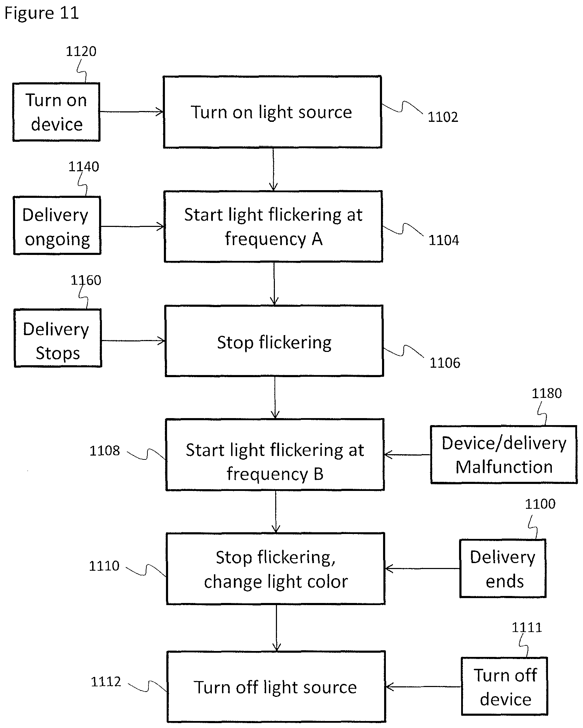

Reference is now made to *FIG. 11* showing a flow chart illustrating an exemplary light indication algorithm, in accordance with some embodiments of the current invention.

In some embodiments, light source is turned on **1102** once the device is turned on **1120,** such as for example when electricity power is sensed by a control circuitry. Optionally, a user can start drug delivering. In some embodiments, while medicament delivery is ongoing **1140,** the light flickers at flickering frequency A **1104.** In some embodiments, when delivery stops **1160,** the light source stops flickering **1106,** optionally returning to the initial operating state. Alternatively the light source emits light having a different color.

In some embodiments, once a malfunction is identified **1180,** the light flickers at frequency B **1108.** In some embodiments, once delivery ends **1100,** light flickering is stopped and optionally light changed color **1110.** In some embodiments, when the device is turned off and/or removed from the user's body **1111,** light is turned off **1112.**

In some embodiments, improper use is also notified by light, such as when a user accidently removes a device while the device is delivering the medicament.

Optionally, a non-visual indication is also provided for any of the device state indications. For example, vibrations, optionally generated by the motor, are provided. Alternatively or additionally, sound indication may be provided for any indication of the device state.

### Exemplary method for providing a user indicator light in a drug delivery device

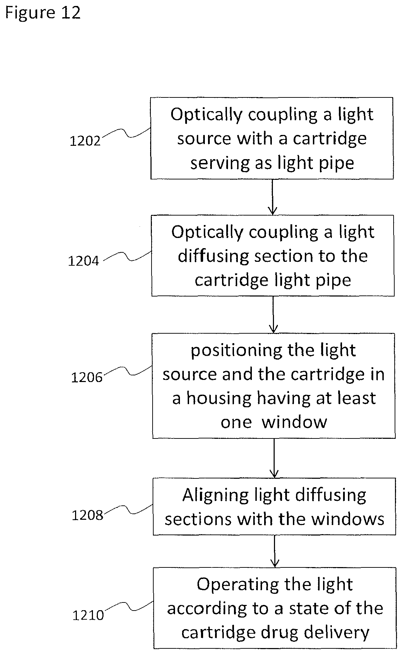

Reference is now made to *FIG.* 12 showing a flow chart illustrating an exemplary method for using a medicament cartridge as a user indicator light, in accordance with some embodiments of the current invention.

In some embodiments, a cartridge containing a medicament is used as a user indicator light by optically coupling a light source with a cartridge configured for serving as a light pipe for light generated by the light source **1202.** In some embodiments, the cartridge further comprises at least one light diffusing section, which is optically coupled to the light pipe of the cartridge **1204,** such that for example, light generated by the light source travels through the light pipe being the cartridge and diffuses out of the cartridge wall through the light diffusing element.

In some embodiments, the cartridge and the light source are positioned in a housing of a drug delivery device having a window **1206.** Optionally, the cartridge and/or light source are aligned with respect to the window **1208,** such that light generated by the light source travels through the light diffusing section and out of the window.

In some embodiments, the light is operated according to a state of the drug delivery device **1210,** in accordance with device states as provided herein.

As used herein the term "about" refers to ± 25 %.

The terms "comprises", "comprising", "includes", "including", "having" and their conjugates mean "including but not limited to".

The term "consisting of' means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

## Claims

1. A pharmaceutical delivery device comprising:
(a) a cartridge containing a fluid, said cartridge including:
(i) a light pipe section which acts as a light pipe having an input location; and
(ii) a light diffusing section optically coupled to said light pipe; and
(b) a light source optically coupled to said cartridge at said input location to said light pipe, such that at least some light from said light source entering at said input location is diffused at said light diffusing section.

2. The pharmaceutical delivery device of claim 1, wherein the light diffusing section is molded with the cartridge in a molding process.

3. The pharmaceutical delivery device of claim 1, wherein a distal end of the cartridge comprises a flange that serves as the light diffusing section.

4. The pharmaceutical delivery device of claim 1, wherein the light diffusing section is a light diffusing surface area configured to diffuse light traveling through the light pipe provided by the cartridge by having a rough surface.

5. The pharmaceutical delivery device of claim 1, wherein said light diffusing section comprises at least one protruding element.

6. The pharmaceutical device according to claim 5, wherein said protruding element at least partially surrounds a circumference of said cartridge.

7. The pharmaceutical device according to claim 5, wherein said at least one protruding element comprises a plurality of protruding elements arranged equidistantly apart along a longitudinal axis of the cartridge.

8. The pharmaceutical device according to claim 5, wherein said at least one protruding element comprises a plurality of protruding elements arranged non-equidistantly apart along a longitudinal axis of the cartridge.

9. The pharmaceutical delivery device of claim 1, wherein said light diffusing section comprises at least one circular bulge.

10. The pharmaceutical delivery device of claim 1, wherein said light diffusing section comprises at least one light diffusing slot.

11. The pharmaceutical delivery device of claim 1, wherein said at least one light diffusing slot is provided along an entire circumference of the cartridge.

12. The pharmaceutical delivery device of claim 1, wherein the light diffusing section is a sticker.

13. The pharmaceutical delivery device of claim 12, wherein the cartridge is lined with the sticker on its inner surface.

14. The pharmaceutical delivery device of claim 12, wherein the cartridge is lined with the sticker over its outer surface.

15. The pharmaceutical delivery device of claim 12, wherein the sticker comprises at least one marking, which comprises text and/or an indicia and/or a symbol.
